Europäisches Patentamt

European Patent Office

Office européen des brevets

(19)

(11) Publication number: **0 186 084 B1**

(12) **EUROPEAN PATENT SPECIFICATION**

(45) Date of publication of patent specification: **04.09.91**   (51) Int. Cl.⁵: **A61K 35/30, C12N 5/00**

(21) Application number: **85116002.8**

(22) Date of filing: **16.12.85**

Divisional application 91101195.5 filed on 16/12/85.

(54) **Brain-derived growth factor.**

(30) Priority: **24.12.84 US 685923**

(43) Date of publication of application:
**02.07.86 Bulletin 86/27**

(45) Publication of the grant of the patent:
**04.09.91 Bulletin 91/36**

(84) Designated Contracting States:
**CH DE FR GB IT LI NL**

(56) References cited:
**US-A- 4 444 760**

**CHEMICAL ABSTRACTS, vol. 100, no. 4, 23rd January 1984, page 85, column 2, abstract no. 30249p, Columbus, Ohio, US; D. GOSPODAROWICZ et al.: "Bovine brain and pituitary fibroblast growth factors: comparison of their abilities to support the proliferation of human and bovine vascular endothelial cells", & J. CELL BIOL. 1983, 97(6), 1677-1685**

(73) Proprietor: **MERCK & CO. INC.**
**126, East Lincoln Avenue P.O. Box 2000
Rahway New Jersey 07065-0900(US)**

(72) Inventor: **Thomas, Kenneth A.**
**245 Washington Avenue
Chatham New Jersey 07928(US)**

(74) Representative: **Abitz, Walter, Dr.-Ing. et al
Abitz, Morf, Gritschneder, Freiherr von Wittgenstein Postfach 86 01 09
W-8000 München 86(DE)**

CHEMICAL ABSTRACTS, vol. 96, no. 13, 29th March 1982, page 109, column 1, abstract no. 116277g, Columbus, Ohio, US; S.C.G. TSENG et al.: "Fibroblast growth factor modulates synthesis of collagen in cultured vascular endothelial cells", & EUR. J. BIOCHEM. 1982, 122(2), 355-360

CHEMICAL ABSTRACTS, vol. 86, no. 7, 14th February 1977, page 320, column 2, abstract no. 40942r, Columbus, Ohio, US; D. GOSPODAROWICZ et al.: "Clonal growth of bovine vascular endothelial cells: Fibroblast growth factor as a survival agent", & PROC. NATL. ACAD. SCI. U. S.A. 1976, 73(11), 4120-4124

CHEMICAL ABSTRACTS, vol. 92, no. 2, 14th January 1980, page 459, column 2, abstract no. 19812n, Columbus, Ohio, US; I. VLODAV-SKY et al.: Vascular endothelial cells maintained in the absence of fibroblast growth factor undergo structural and functional alterations that are incompatible with their in vivo differentiated properties", & J. CELL BIOL. 1979, 83(2), 468-486

CHEMICAL ABSTRACTS, vol. 103, no. 22, 2nd December 1985, page 101, column 1, abstract no. 190072r, Columbus, Ohio, US; K.A. THOMAS et al.: "Pure brain-derived acidic fibroblast growth factor is a potent angiogenic vascular endothelial cell mitogen with sequence homology to interleukin 1", & PROC. NATL. ACAD. SCI. U.S.A. 1985, 82(19), 6409-6413

**Description**

Brain-derived acidic fibroblast growth factor, (aFGF), is an active mitogen for vascular endothelial cells in culture and is useful for growth of such cultures for coverage of polymeric vascular grafts; growth of such cultures on tubular supports for production of blood vessels for implantation; and stimulation or facilitation of blood vessel growth and repair in vivo.

The brain-derived aFGF purification and wound healing activity of this invention was described in U.S. Patent 4,444,760, issued April 24, 1984 to Kenneth A. Thomas, Jr. The protein is also described by Thomas et al., Proc. Natl. Acad. Sci., USA 81, 357-361 (1984). The same or similar proteins may also be present in other partially purified extracts from the central nervous system and other organs. See, for example, Maciag et al., Science, 225, 932 (1984). Endothelial cell growth factor has been applied at a 5000-fold higher concentration than the aFGF concentration of the present invention. See Gospodarowicz, D. et al., J. Cell Biol. 1983, 97(6), 1677-85.

Although the growth of vascular endothelial cells was accomplished in the past using very high concentrations of fetal calf or adult bovine serum (10-30%) the results were variable, depending on the particular lot of calf serum, and the rate of cell growth was generally slow. Now, with the present growth factor rapid growth rates are achieved with serum levels from 0 to 2%.

This novel method of reproducible stimulation of vascular endothelial growth, mediated by pure protein mitogens permits:

1) Covering synthetic polymeric vessels with non-thrombogenic vascular endothelial cells from the host animal, including human, whereby many or all of the clotting problems associated with synthetic vessel grafts are obviated;

2) production of vessels in vitro by growth of host vascular endothelial cells on tubular supports, for implantation back into the same host animal, including human, whereby immunological rejection of the implant will be obviated and the frequent limited supply of good vessels within the patient for transplant will also be obviated.

DETAILED DESCRIPTION OF THE INVENTION

The brain-derived aFGF useful in the novel methods of this invention is prepared as described in U.S. Patent 4,444,760 the disclosure of which is incorporated herein by reference.

The complete amino acid sequence has been determined for aFGF. Sequence determinations of reduced and carboxymethylated protein have revealed two amino termini. The longer sequence, aFGF-1, contains six amino terminal residues not found on the shorter aFGF-2 form. The relative amounts of these two microheterogeneous forms of the mitogen vary from one purification to another but are closely correlated in amount to the abundance of the two bands of protein previously seen by electrophoresis in SDS polyacrylamide gels (Thomas, et al., Proc. Natl. Acad. Sci. USA, 81, 357 (1984)). As expected, the amount of the longer amino terminal sequence correlates with the relative quantity of the higher mass band on the SDS gels. If the length of the polypeptide chain at the amino termini is the only difference between the two microheterogeneous forms observed on the SDS gels, then the mass difference between them is 642 daltons, rather than the previously estimated 200 daltons based on SDS gel migration distances. It is assumed that the amino terminal heterogeneity is the result of limited proteoysis either in vivo or during purification.

The complete amino acid sequence was determined from sequences of the amino termini and overlapping peptides generated by proteolytic cleavages with trypsin (T), Staphylococcus aureus V8 Protease (V8), hydroxylamine (HA) and cyanogen bromide (CN). The carboxyl terminal sequence of the whole protein was confirmed by timed carboxypeptidase A digestion.

In a search of the current Dayhoff protein data bank, aFGF is unique compared to the approximately 2000 protein sequences contained in that list.

The sequence is as follows:

aFGF-1  aFGF-2

1  10

PHE-ASN-LEU-PRO-LEU-GLY-ASN-TYR-LYS-LYS-PRO-LYS-LEU-LEU-TYR-CYS-SER-

T2-4

CNBr-2

HR-6

V8-12

V8-6

20  30

ASN-GLY-GLY-TYR-PHE-LEU-ARG-ILE-LEU-PRO-ASP-GLY-THR-VAL-ASP-GLY-THR-

T2-5  T1-1

CNBr-2

HR-9

V8-12

V8-6

40  50

LYS-ASP-ARG-SER-ASP-GLN-HIS-ILE-GLN-LEU-GLN-LEU-CYS-ALA-GLU-SER-ILE-

T2-7

CNBr-2

HR-9

V8-12

V8-6

60

GLY-GLU-VAL-TYR-ILE-LYS-SER-THR-GLU-THR-GLY-GLN-PHE-LEU-ALA-MET-ASP-

T2-9

CNBr-2

HR-9

V8-1  V8-4

4

Peptide sequences that were prematurely terminated because they were recognized to begin at one of the two previously determined amino termini are marked with asterisks following the last degradation cycle.

The novel method for the in vitro stimulation of vascular endothelial cells comprises treating a sample of the desired vascular endothelial cells in a nutrient medium with aFGF at a concentration of 1-10 ng/ml.

The process requires the presence of a nutrient medium such as Dulbecco's modified Eagle's medium or modification thereof and a low concentration of calf or bovine serum such as about 0 to 2% by volume. Preservatives such as a penicillin-streptomycin combination or other brood spectrum antibacterials are also employed. It is preferred to have about 10 to 100 ug/ml of heparin present also.

The novel method of this invention is useful for the coverage of artificial blood vessels with endothelial cells. Vascular endothelial cells from the patient would be obtained by removal of a small segment of peripheral blood vessel or capillary-containing tissue and the desired cells would be grown in culture in the presence of aFGF and any other supplemental components that might be required such as heparin and/or serum. After growth of adequate numbers of endothelial cells in culture to cover the synthetic polymeric blood vessel the cells would be plated on the inside surface of the vessel. Prior coating of the artificial vessel either covalently or noncovalently, with either heparin or proteins such as fibrin, collagen, fibronectin or laminin would be performed to enhance attachment of the cells to the artificial vascular surface. The cell-lined artificial vessel would then be surgically implanted into the patient and, being lined with the patients own cells, would be immunologically compatible. The non-thrombogenic endothelial cell lining should decrease the incidence of clot formation on the surface of the artificial vessel and thereby decrease the tendency of vessel blockage or embolism elsewhere.

The novel method is also useful for the production of artificial vessels. Vascular endothelial cells and smooth muscle cells from the patient would be obtained and grown separately in culture. The endothelial cells would be grown in the presence of the aFGF as outlined above. The smooth muscle cells would be grown in culture by standard techniques. A tubular mesh matrix of a biocompatible polymer (either a synthetic polymer, with or without a coating of either heparin or specific attachment proteins, or a non-immunogenic biopolymeric material such as surgical suture thread) would be used to support the culture growth of the smooth muscle cells on the exterior side and vascular endothelial cells on the interior surface. Once the endothelial cells form a confluent monolayer on the inside surface and multiple layers of smooth muscle cells cover the outside, the vessel is implanted into the patient.

EXAMPLE 1

MITOGENIC RESPONSE OF FETAL BOVINE THORACIC AORTIC ENDOTHELIAL CELLS TO aFGF

Fetal bovine thoracic aortic endothelial cells (AG4762, N.I.A. Aging Cell Repository, Institute for Medical Research, Camden, New Jersey) were assayed after 38 cumulative population doublings in vitro. The cells were plated in 6-well Costar plates at $2 \times 10^3$ cells/cm$^2$ in 20% heat inactivated fetal calf serum in Dulbecco's modified Eagle's medium (DMEM, Gibco) and changed to 1% serum 18 hours later. All media were supplemented with glutamine and penicillin-streptomycin as previously described. Either pure aFGF diluted in 100 $\mu$l of 1 mg bovine serum albumin (Sigma) per ml of DMEM or serum samples were added to each well along with 1.6 $\mu$Ci of $^3$H-thymidine (New England Nuclear) and 45 $\mu$g of unlabeled thymidine in 40 ul of DMEM. After a 48 hour incorporation period, the cells were washed, lysed and 75% of the trichloroacetic acid (TCA)-insoluble DNA from pure growth factor (-●-) or serum (-■-)-stimulated cells was counted.

The increase in endothelial cell population at various concentrations of aFGF was measured by measuring the uptake of tritiated thymidine. The results are shown in Figure 1, below.

pg aFGF/ml

Figure 1

EXAMPLE 2

MITOGENIC RESPONSE OF MOUSE LUNG CAPILLARY ENDOTHELIAL CELLS TO aFGF

Mouse lung capillary endothelial cells were plated at 2.6 X $10^4$ cells/cm² in 0.5 ml/well in 24-well Costar dishes and grown to confluence in 10% charcoal-treated calf serum (HyClone Laboratories, Logan, Utah) in DMEM, lowered to 0.5% serum after 72 hours and allowed to become quiescent over 48 hours. Either serum or the pure aFGF were added in 50 $\mu$l as described above followed 18 hours later by a 4 hour pulse of ³H-thymidine (20 $\mu$l of 100 $\mu$Ci/ml ³H-thymidine in Gibco phosphate buffered saline). The cells were processed and radioactivity counted as described in Example 1, and the results were as shown in Figure 2.

pg aFGF/ml

**Figure 2**

**Claims**

1. A novel method for the in vitro stimulation of growth of vascular endothelial cells which comprises treating a sample of the desired endothelial cells in a nutrient medium with aFGF at a concentration of 1-10 ng/ml.

2. A method for the stimulation of the growth of vascular endothelial cells in vitro which comprises treating a sample of vascular endothelial cells in a nutrient medium, comprising 2% or less of bovine serum, glutamine and a broad spectrum antibiotic with aFGF at a concentration of 1-10 ng/ml.

3. The method of Claim 2 wherein cells from a vascular explant are grown on the surface of a synthetic polymeric vessel for implanting in the host.

4. The method of Claim 2 wherein endothelial cells from a vascular explant are grown on the interior surface of a biocompatible tubular mesh support and smooth muscle cells are grown on the external surface of the tubular mesh support for implanting in the host.

**Revendications**

1. Nouvelle méthode pour la stimulation in vitro de la croissance de cellules endothéliales vasculaires, comprenant le traitement d'un échantillon des cellules endothéliales désirées dans un milieu nutritif avec de l'aFGF à une concentration de 1-10 ng/ml.

2. Méthode pour la stimulation de la croissance de cellules endothéliales vasculaires in vitro, comprenant le traitement d'un échantillon de cellules endothéliales vasculaires dans un milieu nutritif, comprenant 2% ou moins de sérum bovin, de la glutamine et un antibiotique à large spectre avec de l'aFGF à une concentration de 1-10 ng/ml.

3. Méthode selon la revendication 2, dans laquelle on fait croître des cellules provenant d'un explant vasculaire sur la surface d'un vaisseau polymère synthétique destiné à l'implantation chez l'hôte.

4. Méthode selon la revendication 2, dans laquelle on fait croître des cellules endothéliales provenant d'un explant vasculaire sur la surface intérieure d'un support réticulé tubulaire biocompatible et on fait croître des cellules de muscle lisse sur la surface externe du support réticulé tubulaire destiné à l'implantation chez l'hôte.

**Patentansprüche**

1. Eine neue Methode für die In-vitro-Stimulierung des Wachstums von vasculären Endothel-Zellen, die die Behandlung einer Probe der gewünschten Endothel-Zellen in einem Nährmedium mit aFGF in einer Konzentration von 1-10 ng/ml umfaßt.

2. Eine Methode für die Stimulierung des Wachstums von vasculären Endothel-Zellen in vitro, die die Behandlung einer Probe von vasculären Endothel-Zellen in einem Nährmedium, enthaltend 2 % oder weniger an Rinderserum, Glutamin und ein Breitbandantibiotikum, mit aFGF in einer Konzentration von 1-10 ng/ml umfaßt.

3. Die Methode nach Anspruch 2, worin Zellen eines vasculären Explantats auf der Oberfläche eines synthetischen polymeren Gefäßes für die Implantation in den Wirt gezüchtet werden.

4. Die Methode nach Anspruch 2, worin Endothel-Zellen eines vasculären Explantats auf der inneren Oberfläche eines biokompatiblen rohrförmigen, netzartigen Trägers und glatte Muskelzellen auf der äußeren Oberfläche des rohrförmigen, netzartigen Trägers für die Implantation in den Wirt gezüchtet werden.